# EUROPEAN PATENT APPLICATION

(11) **EP 2 482 062 A1**
(43) Date of publication of application: **01.08.2012**
(21) Application number: 12152697.4
(22) Date of filing: 26.01.2012
(51) Int. Cl.: G01N 25/48, G01N 33/03

(54) **Method for analyzing and identifying edible oils, particularly extra-virgin olive oil**

(30) Priority: 28.01.2011 IT RM20110038
(71) Applicant: Consiglio Nazionale delle Ricerche, 00185 Roma (IT); Azienda Agricola Podere De'Pardi, 56010 Ghezzano San Giuliano Terme (IT)
(72) Inventor: Tombari, Elpidio, 56124 Pisa (IT); Salvetti, Giuseppe, 56124 Pisa (IT); Righetti, Maria Cristina, 56124 Pisa (IT); Pardi, Tommaso, 56010 Ghezzano San Giuliano Terme (IT); Angiuli, Marco, 56124 Pisa (IT)
(74) Representative: Leone, Mario

(57) **Abstract**

A method for analysing and identifying edible oils, particularly extra-virgin oil, can be easily and quickly put into practice and it provides, in a differential calorimeter with temperature scanning, that an oil sample is subjected to a time-temperature protocol, wherein it is provided: a first cooling determining a first solidification of the oil from metastable status of the liquid phase; a first heating determining a partial melting of the oil, so as to leave in the oil residual crystalline structures, therefore in the subsequent cooling the sample solidifies due to the growing of such forms; a second cooling determining a second solidification due to the growing of said residual crystalline structures; a second heating determining the complete melting, the above-mentioned protocol determining a representative thermogram of said sample, which can be compared by means of graphical overlapping with other thermograms in order to determine the matching of the provenance and/or the composition of an additional oil sample.

## Description

The present invention relates to a method for analysing and identifying edible oils, particularly extra-virgin oil, provided with the aim of determining the provenance of the oil itself and the oil belonging to a group of oils identified by the provenance based upon a map of the places of the oil origin.

Such method is then suitable to be used for safeguarding the user as to the controlled origin guarantee and to the repression of possible commercial frauds with particular reference to the extra-virgin oil field.

This method is further suitable to be implemented with low cost and with a simple operating procedure. With this method it is possible creating an identity card of calorimetric type of the oil and implementing a method for mapping the productions and the repression of the commercial frauds.

The most frequent frauds implement by adding fewer expensive oils, such as: groundnut; oleic high sunflower; refined, deodorized, etc., olive oil, in percentages even higher than 20-30% in volume.

The chemical analyses generally are able to reveal such frauds. In some cases (addition of refined kernel) more sensible and expensive techniques are necessary, such as the nuclear magnetic resonance, which allows detecting additions higher than 8-10%. The calorimetry, certainly easier and less expensive technique, showed a greater sensibility for addition percentages of 3-5%.

Currently, the main problem in the field of frauds is the presence on the market of oils with false provenance declaration which can be revealed only with the above-mentioned complex analyses.

The peculiarity of the present analysis method consists in using a time-temperature protocol for scanning differential calorimeter and places in the field of the thermodynamics of fat substances, with particular reference to the analysis of phase transitions of a complex sample under metastable conditions, wherein a suitable definition of the physical conditions, before and after the calorimetric measurement, constitutes a pre-requisite necessary to obtain reproducible data, then data usable for identifying and diagnostic purposes.

The present invention can be applied particularly to the olive oil field, as it takes into consideration the peculiarities of the commercial chain and the needs of the various operators.

Specifically, a preferential application field of the method according to the present invention is the search for and the study of the extra-virgin oil, with reference even to statistics and market analysis, in relation to the olive oil field, and to the quality control and to the repression of frauds.

The state of the art, as to the scientific knowledge therefrom the invention derives and the specific problems of the oil field which the invention contributes to solve, are treated hereinafter.

The extra-virgin olive oil, the Italian one in particular, has an undoubted name and recognition for its quality, the abundance of varietal types and the consequent range of organoleptic features, which allow couplings and specific culinary uses thereof.

To put the extra-virgin oil on the market, the historic-cultural, artistic and landscape resources are resorted to, associated to the description of the product and of the producing firm.

Not last for advertisement effectiveness, the recall to the healthy properties of the extra-virgin oils, especially considered in USA and in Northern Europe.

The ways and means for promoting oil on the market, however, are in continuous evolution. The advertisement onto the printed paper is being ever more replaced by the one by internet, wherein the sites dedicated to the olive oil and the portals for the on-line market are growing. Generally, the oil quality is certified at the origin through chemical analyses and judgements of *panels* of tasters, according to criteria defined by international rules (for example the European regulation) and by the disciplines of the protection consortia.

Nevertheless, the extra-virgin oil market suffers from the poor trust of the consumer who is alerted by recurrent frauds and doubtful for the difficulty of sure controls at the various levels of the industrial and commercial chain. This situation penalizes the producers of high quality oil who, to face the production costs, must go on the market with a considerably higher price than that which the large-scale distributors propose for a product which is only legally defined olive extra-virgin oil.

Furthermore, with the increasing globalization of the markets, the problem of protecting the local productions and the need for fighting the frauds related to the origin of the marketed products increase.

In the light of what above, for several years attempts have been pursuing for creating an oil identity card based upon spectroscopic techniques, but up to now, no results are available in the commercial practice which are easy to be used by the producer and the consumer.

The calorimetry of oils is widely reported in literature, but such technique has never been used to implement an identity card and a conformity and origin card, applicable in the olive oil industry and commerce.

To this purpose, the phase transitions of the edible oils have been for some time considered by the researchers for the complexity of oils, both in liquid phase and in solid phase. The liquid oil is composed by a plurality of triglycerides and organic polar and not polar molecules. The solid oil additionally is formed by polymorphic crystals which, upon varying the temperature, undergo solid-solid transactions, before melting. The spreading of the temperature scanning differential calorimetry (DSC) has made to increase the number of publications on the matter and has underlined the calorimetry potentiality for the study of the oils. The applications shown in literature mainly relate to high temperatures, for industrial applications, such as food frying and the oxidation processes.

Olive oils and the mixtures thereof with oils of raw and refined seeds were studied in a systematic way, by using different calorimetric protocols for the different edible oils and the different purposes of the performed researches, with particular attention to the conditions providing greater sensibility and reproducibility of the results. This allowed to study the effects of the small variations in the chemical composition onto the oil freezing and melting curves.

In particular, the thermogram variations were observed caused by: i) physical methods in the production phase (filtration, squeezing method); ii) chemical methods (refining); iii) addition of oils of different type (mixtures of extra-virgin oil + oil of various seeds; extra-virgin oil + refined kernel oil); extra-virgin + refined olive oil; iv) exposition to the light and oxygen.

The calorimetric analysis has shown to be able to distinguish between extra-virgin oils obtained from different cultivar and even from the same cultivar, even if the fruits have a different ripening levels or come from geographic areas with different pedoclimatic features.

Some of these results have been confirmed by researchers of other laboratories, in Italy and abroad.

From the researches it has resulted that: i) the thermogram is strictly connected to the molecular composition of the oil sample; ii) given the oil complexity, it is practically impossible reducing the whole thermogram of an oil (freezing and melting) with corrective interventions aimed at the sample, then the thermogram represents a kind of digital footprint of the oil. Generally, the aim of the analysis and identification method according to the invention relates to:
● a time-temperature protocol to be used on DSC calorimeter, able to provide, for a given oil sample, the thermogram necessary for: (i) the issue of the identity card of the extra-virgin oil, (ii) the conformity calorimetric test, (iii) the procedure for mapping the productions;
● a method for implementing the mapping of the production by means of a suitable software, by using the calorimetric parameters of a statistically significant number of extra-virgin oils, with certain geographical origin, measured by the thermogram obtained with the protocol of the preceding item, and kept in a data bank;
● a method for issueing an identity card to a *blend* oil obtained by mixing oils from different OEVO lots (blend), commercialized directly by packagers or packaged on behalf of third parties.

In the light of what above mentioned, the known calorimetric methods do not allow implementing a thermogram which is at the same time sufficiently detailed, that is able to identify univocally an oil, and repeatable.

For example, in the article Calorimetry for fast Authentication of Edible Oils published on International Journal of Thermophysics; Journal of Thermophysical Properties and Thermophysics and its Applications dated 30 June 2009 of the same inventors, a calorimetric method is described using two distinct identical cycles, with much higher maximum temperatures than the melting/solidification area and much lower minimum temperatures than the same area. The thermograms which are obtained are not sufficiently representative of the huge complexity of the oil composition, particularly if one relates to the extra-virgin olive oil.

In the light of what above, the technical problem underlying the present invention consists in obviating the drawbacks mentioned by referring to the state of the art.

The solution idea consists in providing to the analysis and identification methods a peculiar heating and cooling protocol so as to exploit different types of phase transition with the aim of obtaining most representative thermograms.

Such technical problem, related to the simple identification of oils and particularly, but not exclusively, extra-vergin olive oils of quality, is then solved by an analysis and identification method as defined in the annexed claim 1.

The main advantage of the present invention consists in providing a method able to allow in a simple way to identify an oil based upon easily processable data.

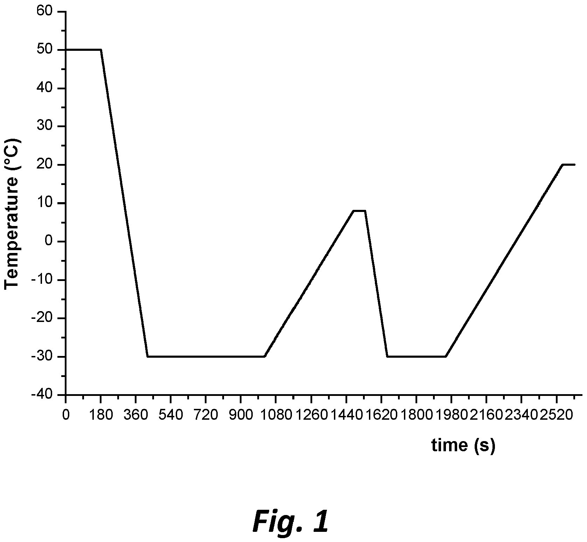

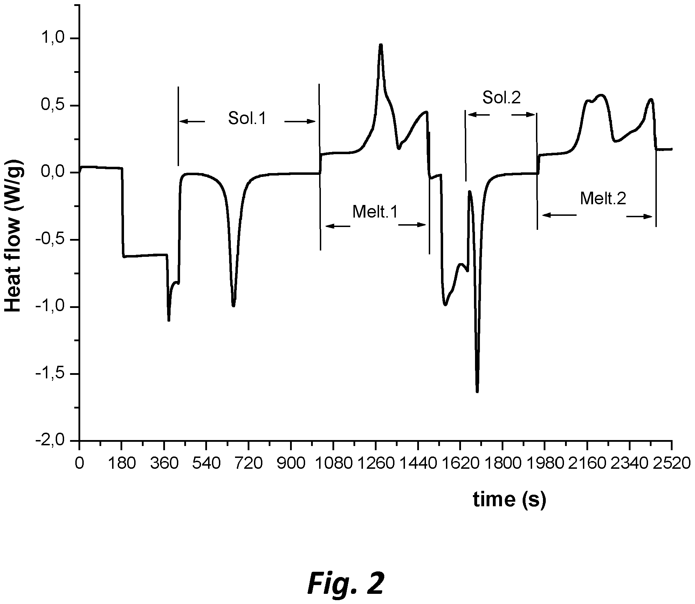

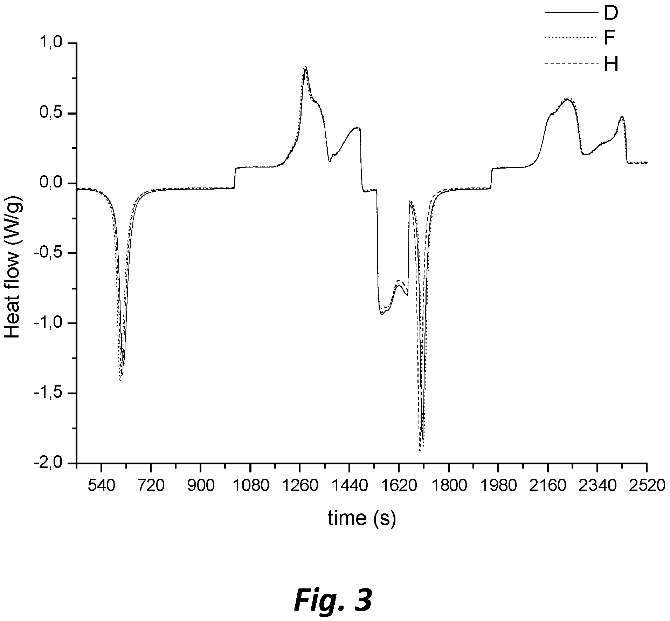

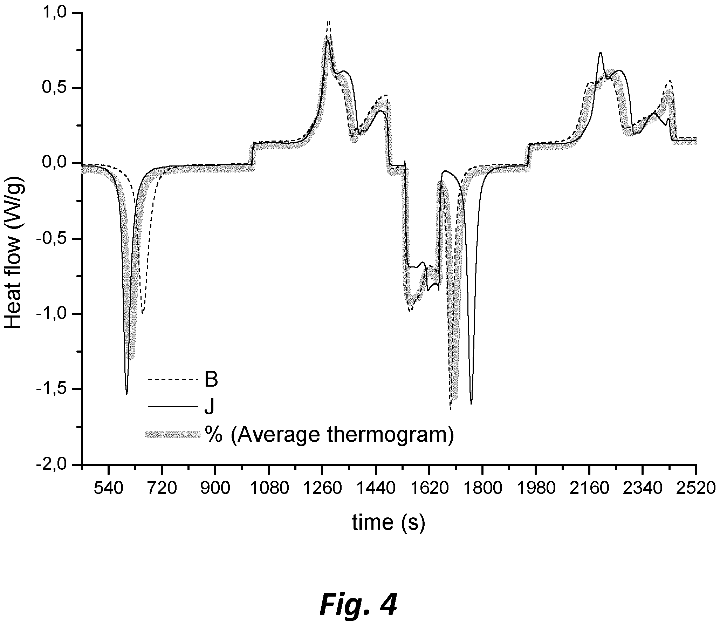

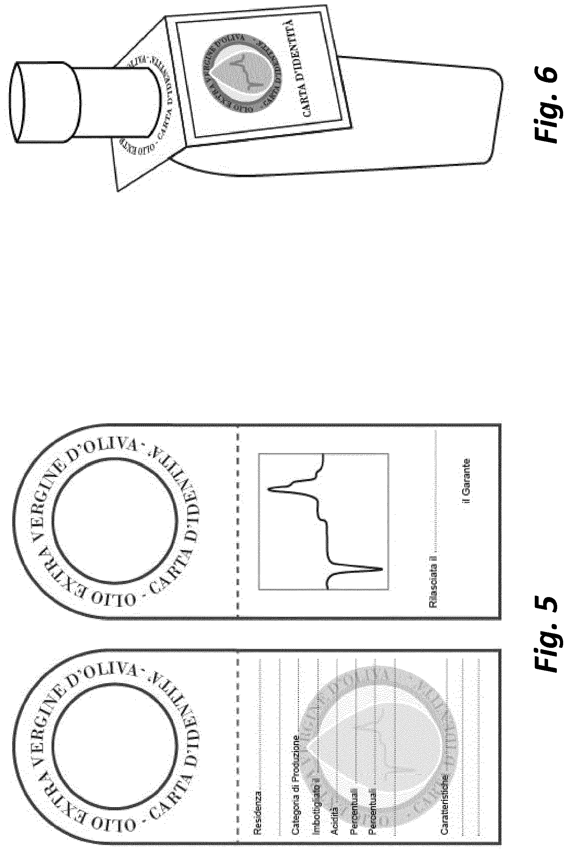

The present invention will be described hereinafter by way of example and not for limitative purpose, by referring to the enclosed figures wherein:
● figure 1 shows the time-temperature protocol to be applied to a sample of extra-virgin olive oil in the method according to the invention;
● figure 2 shows the thermogram obtained by applying the protocol of figure 1 for said oil sample;
● figure 3 shows three distinct thermograms of oils coming from neighbouring geographic areas in an area taken into consideration;
● figure 4 shows a thermogram constituted by a thick curve representing a production map of the area considered in figure 3 and the thermograms of the two oils not selected for the mapping
● figure 5 shows an example of document or identity card to be connected to a specific oil, bearing a thermogram identifying the oil itself; and
● figure 6 illustrates an oil bottle with the document of figure 5 as collar.
● The time-temperature protocol for DSC calorimeter according to the present invention allows obtaining the oil crystallization and melting thermogram under two basically different thermodynamic conditions, both significant for the oil characterization and experimentally reproducible.

On this matter, it has been shown that the phase transitions are particularly sensible to the chemical composition of oils and to the variations thereof. The involved processes, in fact, reflect the oil chemical composition through the interactions between the triglycerides and with the multiplicity of molecules constituting the minority component, regulating the nucleation and growth of the polymorphous crystals.

Therefore, the thermogram, integrated with the chemical-physical analyses and the organoleptic test according to the existing law can be actually considered as identifying a particular extra-virgin oil, with its qualities and geographical provenance.

According to the protocol in figure 1 the sample is kept at 50°C for 3 minutes; then it is cooled at a constant speed of 20° C/min until -30°C. It is kept at this temperature for 10 minutes, then it is heated at the speed of 5°C/min until 8°C. The sample is kept for 1 min. at this temperature, then it is cooled again at the speed of 20°C/min until -30°C. This temperature is kept for 5 min, then it is heated until 20°C, at the speed of 5°C/min. The temperature of 20°C is kept for 3 min.

The whole test period of time is 45.5 min.

For particular applications, such as the conformity test of an oil lot with the sample analysed at the origin, the pieces of information which can be obtained by interrupting the protocol after isotherm at 8°C generally are sufficient. In such case the test time reduces to less than 30 min.

This protocol allows observing, during the first cooling, the OEVO solidification from metastable state of the liquid phase. The molecular interactions determine the nucleation process and the growth kinetics of the polymorphs of triglycerides.

In the second cooling of +8°C, in the melt oil there are residual crystalline structures of type β, therefore the sample solidifies mainly by growth of such shapes.

Upon decreasing the temperature and during the isotherm a -30°C, solid-solid transitions and growth of structures β', stable at low temperatures, intervene. This second oil solidification, then, produces, a polymorphous crystalline sample different from the one obtained in the first cooling, but correlated thereto, as depending from the nature and quantity of residual structures existing in the melt at +8°C, inserted in the same triglyceride matrix.

The subsequent melting curve, in fact, has very different features from the previous one. It has to be underlined that the protocol object of the invention differentiates from what reported in literature, not so much for the cooling and heating speed, as for the sequence of two crystallization-melting cycles of the sample planned to "question" the sample under different but complementary thermodynamic conditions, in order to obtain a kind of "photo" from two different angulations.

Therefore the invention cannot be compared to the simple repetition of the same cycle, which would impose the whole melting and the return to + 50°C before starting the second cycle. This type of protocol, used for testing the thermogram instrumental reproducibility provides the same descriptive parameters. It can neither be compared to the sequence of two cycles different for scanning speed and/or duration of the isotherms as in such case data would be obtained, hardly usable for a better sample characterization. In fact, the thermograms related to the first and second cycle would result to be decorrelated thermodynamically.

Therefore, with the protocol object of the present invention it is possible obtaining in an advantageous and new way a wide set of parameters for further distinguishing an oil from another one with the aim of tracing and identifying the extra-virgin olive oil.

The so-obtained thermogram shows the effects of the interactions of the triglycerides therebetween and with the OEVO minority components, amplified by the sequence:
1. solidification from homogeneous liquid
2. uncompleted melting of the solid
3. solidification in presence of crystalline germ in the melt
4. complete melting.

More generally, in the method according to the invention the two coolings take place at the same constant speed and this is valid even for heatings, in order to obtain complementary and reproducible data.

Furthermore, the absolute value of the cooling speed is higher than the absolute value of the heating speed, in order to grow the details of the melting process.

In order to guarantee a convenient homogeneity of the liquid oil, the first cooling is preceded by an isotherm with higher temperature than room temperature, whereas the coolings quickly reach sensibly low temperatures, thereat the liquid oil is metastable. The second cooling takes place once the first heating is interrupted at +8°C and it is followed y a short isotherm at the sample temperature.

Figure 2 shows the thermogram obtained by applying the protocol of figure 1.

During the first cooling it is observed that the heat flow accompanying a first transition, in a temperature range analogous to that wherein the "cerous transition" of the mineral oils is observed, then the isotherm crystallization at -30°C from metastable liquid. The isotherm duration at -30°C is so as to allow the complete solidification of the oil samples.

During the first heating, the sample undergoes transitions from polymorphous phases to other phases, stable at higher temperature (mainly from β' phases to β phases) and the subsequent melting of most solid portion. The resulting melting curve is characterized by a first peak of complex shape, ending at about 0°C. A second peak of smaller height follows, which at +8°C is near its own maximum value, to designate that the endothermic melting process is almost finished. During the second cooling and the fourth isotherm there is the solidification from germ of the partially melt oil. The nature of the so-grown crystalline polymorphs is shown with greater details by the thermogram registered during the second heating leading to the whole melting of the sample: the first melting peak is substantially different from the one of the first melting, whereas the second peak shows the presence of two well distinct processes.

As "identifying photo" of the sample, to be shown in the data bank and in the documents to identify the oil, the whole curve in figure 2 or part of the same is meant.

The **conformity test** is based upon the comparison of the thermogram of the sample subjected to test with that shown onto the identity document of the original oil, kept in the data bank.

It mainly reduces to a visual and direct comparison, which avoids the use of complex mathematical analyses, not suitable to the needs of the field operators, apart from being little comprehensible for a public of not experts, such as the one thereto one has to revert in a wide programme for promoting the oil on the markets.

In literature time-temperature protocols without the isotherm at -30°C are reported, then based upon temperature scannings in cooling and/or heating. To the research purpose scannings in cooling at 10°C/min until -30°C have been used, followed, after an isotherm at this temperature, by the heating at the same speed until +50°C. For applications of industrial and commercial type this protocol has been considered inadequate.

The descriptive parameters of each single thermogram, obtained with the protocol of figure 1 object of the present patent, in fact, identify in a reproducible way and with greater detail and thus advantageous for the applications the crystallization of the oil sample under different thermodinamic conditions and the subsequent transformation and melting of the polymorphous crystals, grown during the isotherms at -30°C.

The transformation and melting peaks, in fact, are better solved, thanks to the less heating speed of the sample (5°C/min.). The polycrystalline structure of the solid grown from metastable liquid is different from that of the solid grown from germ, as it is clearly shown by the two melting curves. The thermogram resulting from the two different solidification/melting cycles includes a wide number of details identifying the oil and, furthermore, it is enriched with details which are hardly parameterizable, but very useful for the conformity test. The guarantee offered by the identity document, in fact, concretizes through a test based upon the visual comparison of the oil thermogram onto the shelf to the one reported onto the identity document: thermograms which can be overlapped in every detail means that the product contained in the packaging on sale is identical to the original oil.

The **method for mapping the productions** applies to the thermograms in data bank, obtained with the protocol of figure 1. A software is used which considers the parameters characterizing the oil solidification and melting (typically 14 parameters: 5 for the 2 solidifications and 9 for the 2 meltings) to select the similar oils, produced in a same geographical area, homogeneous for pedoclimatic conditions and cultivar types. A suitable software can extract from the data bank the certified oils, of a certain year, produced in the area and having the values of the calorimetric parameters comprised in a range fixed based upon considerations of merit, both scientific (standard deviation of the measurement, intrinsic reproducibility of the processes), and productive one, based upon the descriptive data in memory (collection method, pressing, quality and fruit ripening, percentage of the cultivar composing the *blend,* etc).

After having verified that the so-selected oils have similar chemical and organoleptic analyses, it is useful considering the average of the thermograms of the above-mentioned oils as the thermogram of the typical oil of that area. It will represent the oil reference "photo" for the area identity document. Its chemical-physical and organoleptic features, shown on the area identity document, will be the arithmetical averages of the data bank values for the selected oils.

The area identity documents will be kept in a bank of the production maps. The mapping of the products, repeated for subsequent years, will allow calculating the fluctuations of the area identity document and defining quantity criteria for the oil geographical origin test.

By only way of example of the mapping method, object of the present invention, an application thereof to an area in Toscana is shown, therefrom 5 oils derive, certainly a not sufficient number for a realistic mapping.

Figure 3 shows the thermograms of 3 out of 5 oils, certified by a Consortium with protected geographical indication, produced in the area comprised between three adjacent areas. The 3 oils result to have similar thermograms, whereas the other 2 oils show some differences (see figure 4). The three similar oils most likely derive from olivegroves with the same pedoclimatic features and made with the same cultivar used in practically equal percentages.

According to the mapping method, the average thermogram of such oils is calculated and an uncertainty group is associated to such thermogram, therefore a thick curve is obtained. The curve thickness represents the measurement error (for example the SD calculated by reproducibility test) and the supposable fluctuation of the varietal composition, in case of *blend,* the change in the ripening level of fruits, etc. In other words, a curve is obtained the thickness thereof defines the tolerance range for attributing the origin, to be defined with a systematic research and significant statistics, based upon oils with sure origin and composition.

In order to obtain the area identity document, to be meant as area production map, the average value of the chemical-physical and organoleptic parameters of the oils used to calculate the average thermogram is associated to the average thermogram. Table 1 shows the chemical analyses of the 3 oils of figure 3 and the average to be attributed to the representative oil in the area identity document.

**Table 1**

| | **0469** | **0443** | **0429** | **AVERAGE** |
|---|---|---|---|---|
| Acidity | 0.18 | 0.26 | 0.28 | **0.24** |
| Number of peroxides | 7.6 | 7.9 | 10.1 | **8.53** |
| UV analysis | | | | |
| | | | | |
| K232 | 2.06 | 1.91 | 2.17 | **2.05** |
| K270 | 0.19 | 0.12 | 0.13 | **0.15** |
| DeltaK | 0.002 | 0.001 | -0.001 | **0.00** |
| | | | | |
| Mirystic Acid | 0.01 | 0.01 | 0.01 | **0.01** |
| Palmitic Acid | 13.7 | 13.3 | 13.8 | **13.60** |
| Palmitoleic Acid | 1.2 | 1.1 | 1.2 | **1.17** |
| Heptadecanoic | | | | |
| Acid | 0.1 | 0.1 | 0.1 | **0.10** |
| Heptadecanoic | | | | |
| Acid | 0.1 | 0.1 | 0.1 | **0.10** |
| Stearic Acid | 1.9 | 1.8 | 1.9 | **1.87** |
| Oleic Acid | 74.1 | 74.7 | 73.9 | **74.23** |
| Linoleic Acid | 7.6 | 7.5 | 7.6 | **7.57** |
| Linolenic Acid | 0.7 | 0.7 | 0.7 | **0.70** |
| Arachic Acid | 0.3 | 0.3 | 0.3 | **0.30** |
| Eicosenoic Acid | 0.3 | 0.3 | 0.3 | **0.30** |
| Behenic Acid | 0.1 | 0.1 | 0.1 | **0.10** |
| Lignoceric Acid | 0.1 | 0.1 | 0.1 | **0.10** |
| Elaidic Acid | 0.02 | 0.02 | 0.03 | **0.02** |
| 18:2 + 18:3 | 0.01 | 0.01 | 0.01 | **0.01** |

The thick curve shown in figure 4 shows the production map of the area considered in the present example. The thermograms of the two oils not selected for the mapping are shown too.

These two oils are clearly out of the area production map, as the thermograms thereof are not comprised within the tolerance range. Therefore, they have different origin or they are obtained with fruits of different cultivar.

The method proposes especially for protecting typical productions of high quality and it can be well applied to oils produced with defined varietal types. In case of monovarietal oils the mapping results easier and more effective and the thickness of the area thermogram is certainly reduced.

In each case, by restricting the tolerance range, the production map covers areas with decreasing extension, therefore the definition thereof has to take into consideration the local productive realities, the commercial politics and the purposes which the mapping itself pursue.

The area maps obviously change, year after year, even not substantially. An analysis of the maps of the several years could be used to deepen the knowledge of productions, in particular of those of greater quality.

The issue of the identity card document for *blend* oil could take place as follows.

In the industrial practice, apart from the producer which packages or makes to package his/her own product, the oil mill, the intermediate and the packager operate. The packager and the intermediate purchase oil lots from the producers and/or oil mills, they stow them in silos inside their own factories. With the available oil the packager creates the *blend* oil to be bottled, by mixing, according to company criteria or at customers' request, the several types of oil existing in the firm. The issue of the identity document to the resulting extra-virgin oil, put on the market by this way, *blend* requests a procedure which has to be advantageous for the packager and a real guarantee for the consumer purchasing the product from the shelf.

By taking into consideration the current commercial practice, the following method has been devised:
1) The identity document is issued to each lot purchased in the following manner: the oil sample taken and analysed by the packager is subjected to calorimetric test in the purchase phase, according to the above protocol. Upon the lot delivery, the test is repeated for controlling the conformity of the same to the previously analysed sample. This to protect the packager. The identity control system keeps the oils subjected to test in a sample bank.
2) When the packager create the *blend* oil with the oils equipped with thermogram, he/she communicates to the System Manager the *blend* composition, that is the volume percentages, of oils taken from the various silos and mixed for the bottling.
3) The producer sends a bottle of the resulting *blend* oil to the Manager who proceeds with the colorimetric test. The Manager compares the resulting thermogram to that of the *blend* oil obtained in laboratory, by mixing the components kept in the Sample Bank in the same percentages declared by the packager.
4) If the two thermograms coincide, then the Manager issues the identity document for the *blend* oil, in a number of examples equal to the number of the bottles resulting from the lot of oil to be commercialized.

The described method was tested in the field; the considered *blend* was formed by six different oils and the related thermogram had been obtained from each one thereof. The sample of the *blend* oil provided by the firm gave a thermogram practically coincident with that of the sample obtained in laboratory by mixing the six oils in the same volume percentage.

The application of this method provides a real guarantee for tracing the commercial oils, to protect the consumer.

The present invention implements and applies to the olive oil field through the identity document issued by a System which provides a Manager; a calorimetric laboratory, the sample bank and the related data bank.

The document is issued to the oil packed and put on the market, according to specific procedures for each chain subject (producer, oil mill, packager, distributor, protection body, etc.). It is devised with format and contents suitable to the commercial and economical needs of the chain.

By way of example, a possible operating articulation of said structure is the following:
● the Manager assigns a code to the oil sample received from the operator and he/she sends it to the calorimetric laboratory which performs the thermogram thereof; a portion of the sample is transferred in specific containers, identified too by the assigned code, which will be kept in the sample bank; the thermogram is inserted in the data bank, together with the parameters describing the sample;
● the Manager enters the thermogram codified in the data bank, he/she translates the code and he/she assigns it to the oil sample, now clearly connected to its owner; each subsequent test of conformity of an oil to the original one can be requested to the Manager, who will proceed in comparing the thermogram of the subjected sample to that existing in the data bank;

● alternatively, the applicant can be addressed to the nearest reliable calorimetric laboratory in order to obtain the thermogram according to the protocol provided by the Manager and proceed with the comparison to that of the original one;
● in case of contestation or a product near the expiry, the test will be performed even onto the original oil sample kept in the sample bank, which is the twin being same age of the marketed product.
● The identity document is here described, by only way of example, as a leaflet with four pages (figure 5):
● the front, with the System mark, the indication of the subject belonging to the production chain and the code identifying the identity card corresponding to the packaging;
● the first inner page, with the chemical-physical and organoleptic parameters of the oil, the references of the chain subject, the reference of the examined lot;
● the second inner page, with the "identifying photo", that is the oil thermogram;
● the back page, with the issue data and the instructions for using the document, the addresses of the Manager (postal, e-mail, internet addresses) for information and complaints.

The leaflet, placed in a suitable place, accompanies each package of the product put onto the market (figure 6).

The document includes the oil traditional chemical-physical describers and the organoleptic analysis, provided by the existing law and/or by the protection specifications. These describers are part of the parameters existing in data bank, wherein all pieces of information considered useful for a complete characterization of the product, even for the purpose of research, statistical analyses of the productions and of the market, are kept.

To the above-described method for analysing and identifying edible oils, a person skilled in the art, in order to meet contingent needs, could bring several variants, still within the protection scope defined by the enclosed claims.

## Claims

1. A method for analysing and identifying edible oils, particularly extra-virgin oil, wherein an oil sample is subjected, in a differential calorimeter with temperature scanning, to a time-temperature protocol, providing:
● a first cooling determining a first solidification of the oils from metastable status of the liquid phase;
● a first heating determining a partial melting of the oil, so as to leave in the oil residual crystalline structures, therefore in the subsequent cooling the sample solidifies due to the growing of such forms;
● a second cooling determining a second solidification due to the growing of said residual crystalline structures; and
● a second heating determining a complete melting,
the above-mentioned protocol determining a representative thermogram of said sample, comparable by means of graphical overlapping with other thermograms in order to determine the matching of the provenance and/or the composition of an additional oil sample.

2. The analysis and identification method according to claim 1, wherein said protocol provides:
● a first isotherm at 50°C for 3 minutes;
● a first cooling at constant speed of 20° C/min until -30°C;
● a second isotherm at -30°C for 10 minutes;
● a first heating at constant speed of 5°C/min until 8°C;
● a third isotherm at 8°C for 1 min;
● a second cooling at the constant speed of 20°C/min until -30°C;
● a fourth isotherm at -30°C for 5 min;
● a second heating at the constant speed of 5°C/min until 20°C; and
● a final isotherm at 20°C for 3 min.

3. The analysis and identification method according to claim 1, wherein in said protocol the two coolings take place at the same constant speed.

4. The analysis and identification method according to claim 1, wherein in said protocol the two heatings take place at the same constant speed.

5. The analysis and identification method according to claim 1, wherein in said protocol the absolute value of the cooling speed is greater than the absolute value of the heating speed.

6. The analysis and identification method according to claim 1, wherein in said protocol the first heating and the second cooling are separated by an isotherm comprised between 6° and 10°C.

7. The analysis and identification method according to anyone of the preceding claims, wherein a data bank is constituted with the thermograms obtained by a plurality of sample oils.

8. The analysis and identification method according to claim 7, wherein the thermograms of the oils coming from neighbouring growing areas are used to produce an average thermogram with a thickness so as to contain said thermograms and to represent the oils coming from a determined geographical area.

9. The analysis and identification method according to claim 7, wherein the oil is sold accompanied by an identification document showing its characteristic thermogram, which can be checked by means of said data bank.
